# EUROPEAN PATENT SPECIFICATION

(11) **EP 0 783 330 B1**
(45) Date of publication and mention of the grant of the patent: **01.09.1999**
(21) Application number: 95933005.1
(22) Date of filing: 20.09.1995
(51) Int. Cl.: A61M 1/00, B01D 29/64, B01D 29/37

(54) **DEVICE FOR COLLECTING BONE TISSUE FRAGMENTS**
VORRICHTUNG ZUM SAMMELN VON FRAGMENTEN VON KNOCHENGEWEBE
APPAREIL POUR RECUEILLIR DES FRAGMENTS DE TISSU OSSEUX

(30) Priority: 20.09.1994 SE 9403183
(43) Date of publication of application: 16.07.1997
(73) Proprietor: Bladhs Medical AB, 33010 Bredaryd (SE)
(72) Inventor: LUNDGREN, Dan, 436 51 Hovas (SE)
(74) Representative: Ström, Tore
(86) International application number: PCT/SE95/01065
(87) International publication number: WO 96/09079

(56) References cited:
- EP-A- 0 284 322
- US-A- 3 623 607
- US-A- 3 863 624

## Description

The invention relates to a device for collecting bone tissue fragments from a liquid flow at surgical operations in bone tissue, comprising a tube having axially aligned inlet and outlet sockets at one end and the other, respectively, to be connected into a suction conduit, a cylindrical sieve in the tube, a space being provided between the outside curved surface of the sieve and the inside surface of the tube, an open end of the sieve communicating with the inlet socket of the tube, and said space communicating with the outlet socket of the tube.

A device of this kind is disclosed in EP-A-284 322.

At surgical operations in bone tissue, for example such operations as are carried out in the oral cavity for fixing dental crowns, bridges, and prostheses in a toothless yaw region, drilling is effected in the jaw bone for securing titanium screws which are used as anchoring elements. Often it is in that case desired to recover the bone tissue fragments formed as bone chips at drilling because these can be used for filling the bone cavities formed in the yaw due to preceding pathological processes. By this procedure the jaw bone can be restored in defect regions. At drilling an aspirator is used for removing blood from the site of operation and then the bone tissue fragments will be entrained in the evacuated liquid and will be lost if they are not separated from the liquid flow in some way or other and are collected.

The purpose of the invention is to make this collection possible in a simple way which moreover facilitates the deposition of the bone tissue fragments where desired.

This is achieved by the invention which provides a device according to claim 1.

In order to explain the invention in more detail reference is made to the accompanying drawings in which
FIG. 1 is an axial cross-sectional view of the device according to a preferred embodiment of the invention applied for evacuation of liquid by suction and separation of bone tissue fragments from the liquid,
FIG. 2 is a corresponding axial cross-sectional view of the device applied for deposition of separated and collected bone tissue fragments,
FIG. 3 is an end view of the axially displaceable end wall in the sieve, and
FIG. 4 is a cross-sectional view along line IV-IV in FIG. 1.

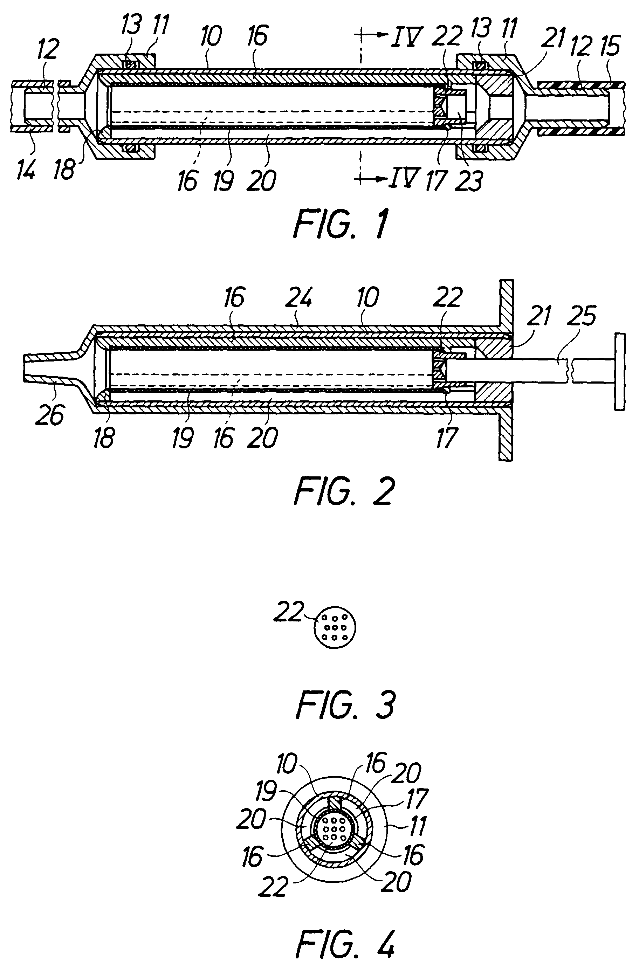

The device according to a preferred embodiment of the invention comprises a cylinder 10 open at each end thereof. At each end a cover 11 is pushed onto the cylinder externally thereof said cover forming a connection piece 12 and being sealed against the outside surface of the cylinder by means of an O-ring 13. An aspirator nozzle 14 is connected to one connection piece, the left one in FIG. 1, said nozzle being shown fragmentarily only. A suction hose 15 is connected to the other connection piece, the right one in FIG. 1, said hose extending to an aspirator not shown herein. The left connection piece with the nozzle thus forms an inlet of the cylinder while the right connection piece with the hose forms an outlet from the cylinder. A cage comprising three straight bars 16 extending in parallel and equally spaced circumferentially, and two rings 17 and 18 integral with and interconnecting said bars, fits detachably in the cylinder, and a cylindrical sieve 19 is received by the cage. By means of the cage the outside curved surface of the sieve is kept spaced from the inside curved surface of the cylinder such that axially extending spaces 20, FIG. 4, are maintained between the sieve and the cylinder between the bars. These spaces are separated from the inlet of the cylinder by means of ring 18 but communicate with the outlet of the cylinder through an annular plug 21 fixed in the cylinder.

At the end of the sieve adjacent the outlet of the cylinder an end wall 22 is provided which at a thicker portion fits against the inside surface of the sieve and at a narrower portion forms a hollow stud 23 which fits displaceably in ring 17 when the wall is in the end position shown in FIG. 1. End wall 22 is axially displaceable along the inside surface of the sieve, and in the embodiment disclosed herein the end wall is perforated although this is not necessary. If it is perforated the apertures should be smaller than the apertures of sieve 19. The device according to FIG. 1 preferably is made in its entirety of plastics and is intended to be a disposable product.

If the device is connected in a suction conduit between the aspirator nozzle 14 and the suction hose 15 as shown herein and if it is assumed that an underpressure is maintained in the suction conduit and that the aspirator nozzle is used for drawing blood from a site in the oral cavity where drilling in the yaw bone is going on, rinsing liquid and blood will be sucked into the sieve via the inlet of the cylinder and will pass through the wall of the sieve to spaces 20 and will continue to the outlet but will also pass through the perforated end wall 22 directly to the outlet. By the underpressure the end wall will be kept in the end position shown; the thicker portion thereof cannot pass through ring 17. If the liquid contains coagulated blood or bone tissue fragments the perforation of end wall 22 will quickly be clogged and occurring solid particles in the liquid will collect on the inside surface of the sieve. However, the perforation of the end wall promotes a direct continuous flow through the device as long as there are no solid particles in the liquid flow.

In order to recover and deposit the material collected in the sieve, cylinder 10 is disconnected from hoods 11 and is placed in a cylinder 24 of an ordinary disposable syringe, FIG. 2, a piston rod 25 then being connected to end wall 22 by the piston rod being inserted into stud 23. The end wall is displaced by means of the piston rod axially through the cylindrical sieve 19 the collected material being scraped off from the inside surface of the sieve wall to be deposited through connection piece 26 of the cylinder, which can be constructed as a Luer connection for connecting a suitable mouth piece to the syringe cylinder. However, it is not necessary to shift cylinder 10 to a syringe for deposition. It is also possible to connect the piston rod to the end wall through the right connection piece 12 in FIG. 1 and to deposit the material through the left connection piece 12 in FIG. 1. It may be comfortable, however, to operate a syringe at deposition.

## Claims

1. Device for collecting bone tissue fragments from a liquid fluid flow at surgical operations in bone tissue, comprising a tube (10) having axially aligned inlet and outlet sockets (12) at one end and the other, respectively, to be connected into a suction conduit, a cylindrical sieve (19) in the tube, a space (20) being provided between the outside curved surface of the sieve and the inside surface of the tube, an open end of the sieve communicating with the inlet socket of the tube, and said space communicating with the outlet socket of the tube, **characterized** in that a piston (22) displaceably received in the sieve and engaging the inside curved surface thereof forms an end wall in the sieve at the outlet socket end thereof, and that a piston rod (25) is insertable into the tube through the outlet socket thereof to engage the piston for manual axial displacement thereof through the sieve under deposition of bone fragments trapped by the sieve, through the inlet socket of the tube.

2. Device as in claim 1 **characterized** in that the piston (22) has a hollow stud (23) for the connection of the piston rod (25).

3. Device as in claim 1 or 2 **characterized** in that the tube (10) at the ends thereof is provided with end covers (11) forming the inlet and outlet sockets, respectively, for the connection of an aspirator nozzle (14) and a suction hose (15), respectively.

4. Device as in any of claims 1 to 3 **characterized** in that the piston (22) is perforated the apertures thereof being narrower than the apertures of the sieve.

5. Device as in any of claims 1 to 4 **characterized** in that the sieve (19) is mounted in a cage fitting in the tube (10) and having axially extending bars (16) which form spacers between the sieve and the tube.

## Patentansprüche

1. Vorrichtung zum Sammeln von Knochengewebefragmenten aus einem flüssigen Fluidstrom bei chirurgischen Eingriffen in Knochengewebe mit einem Rohr (10), das an dem einen und an dem anderen Ende jeweils axial ausgerichtete Ein- und Auslaßstutzen (12) aufweist, um an eine Ansaugleitung angeschlossen zu werden, einem zylindrischen Sieb (19) in dem Rohr, wobei ein Raum (20) zwischen der gekrümmten Außenfläche des Siebs und der Innenflache des Rohrs vorgesehen ist, ein offenes Ende des Siebs mit dem Einlaßstutzen des Rohrs verbunden ist und der Raum mit dem Auslaßstutzen des Rohrs verbunden ist, **dadurch gekennzeichnet**, daß ein Kolben (22), der verschiebbar in dem Sieb vorgesehen ist und mit dessen gekrümmter Innenfläche in Eingriff steht, eine Endwand in dem Sieb an dessen Auslaßstutzenende bildet, und daß eine Kolbenstange (25) in das Rohr durch dessen Auslaßstutzen einsetzbar ist, um mit dem Kolben in Eingriff zu kommen und diesen unter Entfernung der Knochenfragmente, die in dem Sieb gefangen wurden, durch den Einlaßstutzen des Rohrs manuell in axialer Richtung durch das Sieb zu schieben.

2. Vorrichtung nach Anspruch 1, **dadurch gekennzeichnet**, daß der Kolben (22) einen hohlen Ansatz (23) für die Verbindung mit der Kolbenstange (25) aufweist.

3. Vorrichtung nach Anspruch 1 oder 2, **dadurch gekennzeichnet**, daß das Rohr (10) an seinen Enden mit Endabdeckungen (11) versehen ist, die die jeweiligen Einund Auslaßstutzen bilden, um entsprechend eine Ansaugdüse (14) und einen Ansaugschlauch (15) anzuschließen.

4. Vorrichtung nach einem der Ansprüche 1 bis 3, **dadurch gekennzeichnet**, daß der Kolben (22) perforiert ist, wobei seine Löcher engmaschiger als die Löcher des Siebes sind.

5. Vorrichtung nach einem der Ansprüche 1 bis 4, **dadurch gekennzeichnet**, daß das Sieb (19) in einem Gehäuse angebracht ist, das in das Rohr (10) eingesetzt ist und sich in axialer Richtung erstreckende Stangen (16) aufweist, die Abstandshalter zwischen dem Sieb und dem Rohr bilden.

## Revendications

1. Dispositif pour recueillir des fragments de tissus osseux à partir d'un écoulement fluide de liquide, lors d'interventions chirurgicales dans des tissus osseux, lequel dispositif comprend un tube (10) présentant des éléments de raccordement d'entrée et de sortie (12) en alignement axial et respectivement situés à une extrémité et à l'autre extrémité, pour une connexion à un conduit d'aspiration, un tamis cylindrique (19) à l'intérieur du tube, un espace (20) étant prévu entre la surface extérieure courbe du tamis et la surface interne du tube, une extrémité ouverte du tamis communiquant avec l'élément de raccordement d'entrée du tube, et ledit espace communiquant avec l'élément de raccordement de sortie du tube, **caractérisé** en ce qu'un piston (22), logé de manière mobile dans le tamis et venant en contact avec la surface intérieure courbe de celui-ci, forme une paroi d'extrémité dans le tamis au niveau de l'extrémité de celui-ci du côté élément de raccordement de sortie, et en ce qu'une tige de piston (25) peut être introduite dans le tube par l'intermédiaire de l'élément de raccordement de sortie de celui-ci pour venir en contact avec le piston pour un déplacement axial, à commande manuelle, de celui-ci dans le tamis lors du dépôt de fragments d'os, arrêtés par le tamis, par l'intermédiaire de l'élément de raccordement d'entrée du tube.

2. Dispositif selon la revendication 1, **caractérise** en ce que le piston (22) comporte un ergot creux (23) pour la connexion de la tige de piston (25).

3. Dispositif selon la revendication 1 ou 2, **caractérisé** en ce que le tube (10) est pourvu, au niveau de ses extrémités, de capuchons d'extrémité (11) constituant les éléments de raccordement d'entrée et de sortie, respectivement, pour le montage respectif d'une buse aspirante (14) et d'un flexible d'aspiration (15).

4. Dispositif selon l'une des revendications 1 à 3, **caractérisé** en ce que le piston (22) est perforé, ses ouvertures étant plus étroites que ne le sont les ouvertures du tamis.

5. Dispositif selon l'une des revendications 1 à 4, **caractérisé** en ce que le tamis (19) est monté dans une cage s'adaptant dans le tube (10) et comportant des barrettes (16), qui s'étendent axialement et qui constituent des pièces d'écartement entre le tamis et le tube.
